# EUROPEAN PATENT SPECIFICATION

(11) **EP 2 534 493 B1**
(45) Date of publication and mention of the grant of the patent: **11.12.2019**
(21) Application number: 11706637.3
(22) Date of filing: 14.02.2011
(51) Int. Cl.: G01N 35/00, C12M 1/26, C12Q 1/04, C12Q 1/06, G01N 1/31, G01N 1/40, G01N 15/06

(54) **AUTOMATIC TOXICOLOGICAL FLUID SAMPLE PREPARATION MODULE, AUTOMATIC ANALYSIS SYSTEM AND METHOD FOR USE THEREOF**
MODUL ZUR AUTOMATISCHEN TOXOLOGISCHEN FLÜSSIGKEITSPROBENNAHME, AUTOMATISCHES ANALYSESYSTEM UND VERFAHREN ZU SEINER VERWENDUNG
MODULE DE PRÉPARATION AUTOMATIQUE D'ÉCHANTILLONS TOXICOLOGIQUES FLUIDES, SYSTÈME D'ANALYSE AUTOMATIQUE ET PROCÉDÉ POUR SON UTILISATION

(30) Priority: 06.04.2010 WO PCT/NL2010/050177; 12.02.2010 NL 2004239
(43) Date of publication of application: 19.12.2012
(73) Proprietor: Dutch Water Technologies B.V., 8934 CJ Leeuwarden (NL)
(72) Inventor: JANSEN, Gijsbert Johan, NL-9061 DK Giekerk (NL)
(74) Representative: Verdijck, Gerardus
(86) International application number: PCT/NL2011/050104
(87) International publication number: WO 2011/099862

(56) References cited:
- WO-A2-01/46382
- JP-A- 4 030 798
- US-A- 3 940 250
- US-A- 5 627 042
- US-A- 5 801 052
- US-A- 5 801 052
- US-A- 5 821 066
- US-A- 6 007 235
- US-A1- 2003 092 170
- US-A1- 2004 241 828
- US-A1- 2005 064 599
- US-A1- 2006 257 941
- US-A1- 2007 231 844
- US-A1- 2009 111 167
- US-B1- 6 312 943

## Description

The present invention relates to an automatic toxicological fluid sample preparation module for automatically preparing a fluid sample. More specifically, the module is used for preparing a fluid sample for detection and measurement of toxics and/or toxicological effects of substances. A toxic may for example comprise mercury, medication, poison, a toxin such as a neurotoxin, venoms, disinfectants or radioactive substances.

US 2006/257941 discloses a method and device for detection of analytes in a fluid with a portable device.

US 5,801,052 discloses an aqueous sample testing apparatus that specifically relates to measuring the toxicity of polluted water.

In practice, detection and measurement of toxics or toxicological effects is performed by taking a sample from a fluid or fluid stream and subsequently measuring and analyzing the sample in a laboratory. Each sample has to be handled manually, at least to some extent, which is labor intensive and slow.

Furthermore, in practice, the act of obtaining the sample may expose the laboratory worker in certain cases to unnecessary risks. Also, the analysis has to be performed by skilled personnel in specialized laboratories. This is a problem in non-clinical settings where detection and measurement of toxics and/or toxicological effects is important.

The object of the present invention is therefore to provide an effective and efficient fluid sample preparation module for automatically preparing a fluid sample, specifically for subsequent detection and measurement of toxics and/or toxicological effects.

This object is achieved with the automatic toxicological fluid sample preparation module for automatically preparing a fluid sample for the detection and measurement of toxics and/or toxicological effects according to claim 1.

Detection of toxics and/or toxicological effects may comprise establishing whether toxics and/or toxicological effects are present. Measuring may comprise detecting and/or quantifying. Analyzing may also comprise measuring, detecting and/or quantifying.

The module can be provided as a stand-alone system for preparing a fluid sample or in combination with measurement means.

By providing an inlet for automatically obtaining a sample directly from a fluid or fluid stream to be analyzed, the module can run autonomously. At any given time the module can automatically obtain a sample directly from a fluid or fluid stream to be analyzed and prepare a fluid sample from the sample.

For example, the fluid or fluid stream comprises water, urine, sugar syrup or a beverage such as beer.

A sample of a fluid or fluid stream is obtained through the inlet. The preparation means obtain this sample through the first coupling means and prepare a fluid sample from the sample. Optionally, the preparation means comprise a solvent container and a pump to add a solvent to the sample. Further examples of preparation means include: a filter, a heater, a cooler, mixing means, pumps for pumping fluids within the module, indicator micro-organisms which can be added to the fluid sample and nutrition for the indicator micro-organisms which can also be added to the fluid sample.

An advantage of the module according to the invention is that it automatically obtains a sample from a fluid or fluid stream to be analyzed. The invention provides a so-called in-process system: the module has direct access to a fluid or fluid stream of a process for preparation of a fluid sample for subsequent measurement and detection. For example, this enables obtaining a sample from a fluid or a fluid stream at a remote location and/or located in a hazardous environment.

A further advantage is that the system enables a periodical analysis of a fluid or fluid stream by taking samples at different times.

In addition, it is possible to increase statistical accuracy by performing an analysis on a set of samples obtained from the same fluid or fluid stream.

An even further advantage is that the module can be configured to automatically obtain samples from a plurality of fluids or fluid streams. Since the system is in-process and automatic, mistakenly interchanging fluid samples is avoided.

Furthermore, the system is portable, which is for example advantageous in situations in which automatic analysis is required at different locations at great distances from each other.

Therefore, the module according to the invention enables an effective and efficient preparation of a fluid sample for the detection and measurement of toxics and/or toxicological effects.

In a preferred embodiment according to the present invention, the module comprises communication means. The communication means can for example comprise GPRS, infrared, cables or other wired or wireless communication means. Furthermore, the communication means may comprise control signals for controlling the module. For example, the control signals can be generated on the basis of subsequent measurement and analysis of a fluid sample. This enables so called sample exploration, in which information obtained on the basis of a measurement is used to alter the preparation procedure to increase measurement accuracy and precision.

For example, if a measurement on a prepared fluid sample reveals that the total amount of indicator micro-organisms was too low to provide a good measurement, automatic preparation module can be instructed to add more indicator micro-organisms in the next sample.

An advantage of the communication means is that it enables remote control of the module. This is especially advantageous in situations in which the fluid or fluid stream to be analyzed is located remotely, located in a hazardous environment or is otherwise difficult to access.

According to the present invention the indicator micro-organisms emit light and/or produce a light emitting substance, intrinsically and/or in response to a toxic and/or toxicological effect.

The indicator micro-organisms may for example emit bioluminescence light or produce a fluorescent protein. As another example, a genetically modified micro-organism may start producing a fluorescent protein in response to DNA damage brought about by a mutagen.

The module adds indicator micro-organisms to the fluid sample using the preparation means. The indicator micro-organisms have light emitting, absorbing and/or reflecting properties or they produce a light-emitting substance. The emitted light can be detected and quantified in a subsequent measurement of the prepared fluid sample using appropriate measurement means. Toxics and/or toxicological effects may directly or indirectly influence the light properties of the indicator micro-organisms, which can be detected and quantified using the measurement means. A deviation of the light measurements from the expected values indicates the presence of one or more toxics and/or toxicological effects.

An advantage of the measures of this preferred embodiment is that they enable the detection of the toxicological effect of the sample. In present practice, detection and measurement of toxics often focuses on the detection and measurement of specific toxics. The present invention instead prefers to measure the toxicological effect. This enables the detection and quantification of toxicity and toxicological effect, even when it is not known which toxics can be expected. In other words, establishing whether a fluid or fluid stream induces a toxicological effect is considered more important than specifying which specific toxic causes this effect.

It is noted that the present invention may also include detection and measurement of a specific toxic, for instance by providing a micro-organism which is substantially sensitive to the specific toxic only.

For example, according to the invention intrinsically light-emitting indicator micro-organisms are added in a preparation step to a fluid sample. Some indicator micro-organisms are affected by a toxic and the intensity of the emitted light decreases. Therefore, a decrease in light intensity can be detected by measurement means in relation to an expected light intensity, indicating toxicity of the fluid sample.

Different types of toxicological effects can be classified. For example, membrane damage, mutagenicity and metabolism damage. Examples of ways of detecting and measuring these different classes of toxicological effects are presented below.

Preferably, the indicator micro-organisms are Genetically Modified Organisms (GMOs) which have been modified such that one or more light properties, e.g. color, fluorescence or luminescence, is altered or effectuated in response to a toxic and/or toxicological effect. This change in light property is proportional to the toxic or toxicological effect and can be detected by measurement means.

Membrane damaging toxics damage the membranes of cells of the indicator micro-organisms. Healthy cells are not permeable for DNA-coloring reagents. If the membrane of the cells is damaged, for example due to a toxic, DNA-coloring reagents can enter the cells, thereby coloring the cells. The color of the cells can be detected and quantified using measurement means. By using DNA-coloring reagents and appropriate indicator micro-organisms in the module according to the present invention to prepare a fluid sample, membrane damaging toxics can be detected and measured subsequently.

Mutagens damage DNA of the indicator micro-organisms. For example, this can induce cancer. Carcinogens can be detected and quantified, for example, by adding a GMO which responds to DNA damage by emitting light. The module according to the invention can add such GMOs to a fluid sample. The light emitted in response to DNA damage can be detected using appropriate measurement means.

Metabolism damage can be detected and measured by applying the principle that certain coloring agents can be, at least partly, converted or removed by healthy cells. However, when the cells suffer from metabolism damage, the coloring agents will be converted or removed to a lesser extent and the cells will become more colored. The module according to the invention enables adding micro-organisms and coloring agents to a fluid sample for subsequent measurement according to this principle using appropriate measurement means.

According to the present invention the indicator micro-organisms are locked in the log-phase of multiplication before use.

Indicator micro-organisms multiply exponentially under the right conditions. These conditions for example comprise pH-level, temperature, oxygen level and availability of nutrition. It is possible to lock indicator micro-organisms in the exponential phase (log-phase) of growth by quickly removing nutrition, oxygen and lowering the temperature. The indicator micro-organisms are "trapped" in the log-phase until they again are exposed to the right conditions, e.g. they have access to nutrition. A suitable preferred medium for preservation of indicator micro-organisms locked in the log-phase is glycerol, for example. In glycerol, the indicator micro-organisms are deprived from nutrition and oxygen, which ensures that they stay locked in the log-phase.

It is noted that although cooling is used to lock the indicator micro-organisms in the log-phase of multiplication, cooling is not per se required for storing the indicator micro-organisms. Even at room temperature, the indicator micro-organisms can be stored for at least months. Optionally, cooling means are provided in the module according to the present invention, which enables storage of the indicator micro-organisms of years.

An advantage of using indicator micro-organisms which are locked in the log-phase of multiplication before using them in the fluid sample is that the exponential growth will continue when the indicator micro-organisms are again exposed to appropriate nutrition and/or oxygen. Therefore, in a relatively short amount of time, typically less than a few minutes or an hour, sufficient indicator micro-organisms will be available for the measurement and detection.

According to the present invention, the preparation means further comprise nutrition for indicator micro-organisms for adding the nutrition to the indicator micro-organisms.

By adding nutrition, indicator micro-organisms which are locked in the log-phase of multiplication are "unlocked" and continue to multiply. After an initial phase, the so-called lag-phase, the multiplication continues exponentially. Therefore, in a relatively short amount of time, typically less than a few minutes or an hour, sufficient indicator micro-organisms will be available for the measurement and detection. For example, the nutrition is added to the indicator micro-organisms before adding them to the fluid sample or added directly to the fluid sample in which the indicator micro-organisms are subsequently added or have been added in advance. Optionally, the nutrition comprises oxygen. It is noted that nutrition can also be added to micro-organisms which are not locked in the log-phase.

Conventional methods for increasing the number of indicator micro-organisms often involve a step of pre-incubation. Due to the step of pre-incubation, these methods require considerable time for carrying out, usually at least 24 to 48 hours, and generally are labor consuming. The module according to the invention provides a way for increasing the number of indicator micro-organisms to enable subsequent detection thereof which is faster, automatic and autonomous.

According to the present invention, the preparation means comprise a light source for irradiation of the micro-organisms, wherein the light source emits light of a wavelength which stimulates the multiplication of the micro-organisms.

When indicator organisms are unlocked from their dormant state in the lag-phase, it takes a certain amount of time before they start multiplying. This amount of time can be decreased by irradiating the micro-organisms with an appropriate wavelength. The light stimulates multiplication.

For prokaryotic micro-organisms, the appropriate wavelength typically is in the red region of the visible spectrum, for instance 630 nm. For eukaryotic micro-organisms, the typical wavelength is in the green spectrum.

It is noted that also a heater can be used to perform a similar function.

According to the present invention, the module further comprises killing means for killing the indicator micro-organisms. Regulations prohibit releasing GMOs in the environment. Therefore, when the indicator micro-organisms in the system according to the invention are GMOs, the indicator micro-organisms can not simply be discharged as waste. The killing means provide a solution by killing the indicator micro-organisms before discharging them to waste, thereby ensuring that no living GMOs enter the environment.

Naturally, the killing means can also be used to kill other micro-organisms than indicator GMOs, if desired.

According to the present invention the inlet and the preparation means comprise:
a first inlet for obtaining a sample from a fluid or fluid to be analyzed;
a fluid chamber connected to the first inlet; fluid sample preparation means comprising one or more of the following components: a filter, a heater, a cooler, a pump, a piston and mixing means; and
a first outlet for discharging a prepared fluid sample, the first outlet being connectable to measurement means.

For example, the fluid chamber is provided as a fluid container. Preferably, the fluid chamber is provided as a conduit system comprising a reactor.

The fluid chamber obtains a sample from a fluid or fluid stream through the first inlet. A fluid sample is prepared using the fluid sample preparation means. Optionally, a filter is provided for filtration of the fluid sample. A heater can be provided for heating the fluid sample, a cooler can be provided for cooling the fluid sample, a pump can be provided for example for pumping fluid in and out the fluid chamber, a piston can be provided for example for pumping fluid through the filter and mixing means can be provided for mixing the fluid sample.

Embodiments of the invention may include a single element of the listed fluid sample preparation means, or a combination of these elements. The prepared fluid sample can be discharged through the first outlet, which is connectable to the measurement means. In this manner, the prepared fluid sample can be transported to measurement means for subsequent measurement.

It is noted that a piston can function as a fluid chamber.

In an optional embodiment according to the present invention the module further comprises a filter, a second inlet for adding indicator micro-organisms, a third inlet for adding a solvent and/or displacement fluid and a second outlet for discharge of fluids to a waste container, wherein the second inlet is provided at the same side of the filter as the fluid chamber.

A solvent can be added using the third inlet. For example, this enables dilution of the fluid sample.

A displacement fluid can be added using the third inlet. This enables displacement of the fluids present in the system. For example, the fluids can be displaced to the second outlet for discharge of the fluids to a waste container.

The second inlet is provided at the same side of the filter as the fluid chamber. Indicator micro-organisms can not pass the filter. Indicator micro-organisms which are added through the second inlet, therefore remain in the fluid chamber, even when the fluid is moved through the filter to, for example, the waste container. This has the advantage, that even after adding the indicator micro-organisms to the fluid sample, other preparation steps can be performed.

The second outlet for discharge of fluids to waste can be provided at many different positions in the module. For example, it may be connected to the preparation means.

Optionally, the inlet is provided at the opposite side of the filter in relation to the fluid chamber. This enables filtering out undesired contents from the fluid or fluid stream, so that these will be absent in the fluid sample.

In a preferred embodiment according to the present invention the fluid sample preparation means comprise a modular container system, comprising one or more containers for holding processing fluids, indicator micro-organisms, nutrition and/or fluorescent beads.

The processing fluids may for example comprise cleaning fluids for cleaning the different parts of the system, DNA coloring agents or a buffer fluid, such as purified water.

The fluorescent beads have a known light emitting property. By preparing a fluid sample containing fluorescent beads, a reference for analysis of subsequent measurements is obtained. The fluorescent beads enable a calibration procedure.

An advantage of the modularity of the container system is that it enables adding, replacing or removing containers holding different indicator micro-organisms, processing fluids, nutrition and/or fluorescent beads. Depending on the requirements of the measurement and analysis of a certain process, suitable indicator micro-organisms, processing fluids, nutrition and/or fluorescent beads can be selected. The automatic fluid sample preparation module is therefore multi-purpose and can be used in multiple situations. A further advantage is that the containers can be easily replaced when empty or broken.

The fluid sample preparation means can add a single type of indicator micro-organism, or multiple types of indicator micro-organisms, to the fluid sample. Adding multiple types of indicator micro-organisms enables, for example, a measurement in which both a carcinogenic effect and a membrane damaging effect are detected and quantified.

For example, the filter comprises plasma-polished stainless steel, wherein the filter comprises holes having a diameter in the range of 0 - 1 µm.

By providing the filter of stainless steel, a high strength is ensured. The fact that the filter comprises plasma-polished stainless steel ensures that the filter is easy to clean and no, or relatively less, precipitate or deposit is formed on the filter. A filter can therefore be re-used after a simple cleaning procedure. The cleaning procedure can for example be performed using a conventional dish-washer.

By providing holes having a diameter in the range of 0 - 1 µm, the filter is optimal for filtering out micro-organisms, which typically have dimensions larger than 0.5 µm. Preferably, the holes have a diameter of 0.5 µm.

The invention further relates to an automatic toxicological analysis system comprising the fluid sample preparation module as described above and a second module comprising measurement means, preferably comprising a light detector.

The same effects and advantages apply in respect to the analysis system as those described in respect to the automatic toxicological fluid sample preparation module.

Preferably, the measurement means comprise coupling means for coupling them to the outlet of the preparation module to obtain a prepared fluid sample.

Preferably, the measurement is carried out using a light detector. The detector can for example detect and measure fluorescent light, (bio)-luminescent light, direct light and reflected light.

Preferably, the system comprises communication means, for example for remote control of the module. Further examples include a display, a light signal or a sound signal for communicating an alarm, for example.

An advantage of the communication means is that by generating a control signal, such as for example an alarm signal or a process control signal, the system can effectuate process control actions on the basis of the in-process measurement and analysis. This enables a relatively fast response to analysis results. This is especially advantageous in cases in which an undesired or harmful amount of toxics is detected.

In a preferred embodiment according to the present invention, the automatic toxicological analysis system comprises a first module comprising the inlet and preparation means, and a second module comprising the measurement means.

Providing a modular system improves the flexibility of the system according to the invention. A module of the system can be replaced or altered without affecting the other modules in the system. For example, this may involve and improve repairs, maintenance and cleaning of the modules.

In case other characteristics of the system are required, the modularity of the system allows for an update of only those modules that have been altered.

The modularity further has the advantage that specific modules can be added or removed, depending on the fluid or fluid stream to be analyzed. Moreover, the modules can even be used separately. For example, in a situation in which the sample of the fluid or fluid stream does not require automatic preparation, the module comprising the measurement means can operate stand-alone.

In a preferred embodiment according to the invention, the measurement means comprise:
- a light detector; and
- a cuvette with a length, the cuvette comprising:
- an inlet for obtaining the fluid sample; and
- an outlet for discharging the fluid sample.

By providing a cuvette with an inlet for obtaining the fluid sample and an outlet for discharging the fluid sample, it is possible to automatically obtain the fluid sample from the preparation means and discharge this fluid sample after the measurement and analysis.

In a preferred embodiment according to the present invention the cuvette comprises flushing means. This has the advantage that the same cuvette can be used for several measurements. A further advantage is that the measurements will be clean, i.e. the fluid sample being measured is not polluted with a previous fluid sample.

Preferably, one or more of the light detectors comprise Charge Coupled Devices (CCDs) provided in an array extending substantially over the length of the cuvette.

Using a CCD as a light detector has the advantage of a large dynamical range and a high quantum efficiency. The CCD is therefore able to detect both very low light levels and very high light intensities.

The advantage of using a CCD provided in an array extending substantially over the length of the cuvette is that the detection area is maximized. Light from the fluid sample is emitted in every direction. It is therefore advantageous to maximize the detection area, especially in the case of low light intensities.

In a preferred embodiment according to the invention, the measurement means further comprises a light source.

The light source enables irradiation of the fluid sample, for example to enable detection of indicator micro-organisms of a certain color or to induce fluorescent light emission in indicator micro-organisms or substances produced by indicator micro-organisms, such as proteins.

The light source further enables an absorption measurement.

Preferably, the light source comprises a light emitting diode (LED). Advantages of LEDs include low costs, relatively high intensity, low energy consumption, narrow emission bandwidth and a stable intensity over time. For example, an ultraviolet LED can be provided.

Optionally, more than one light source is provided to increase the total intensity of the light or to irradiate the fluid sample with light of different wavelength and/or intensity, for example.

Preferably, according to the invention, a light detector is provided at an angle of substantially 90 degrees with respect to the light source.

By providing the light detector at an angle of substantially 90 degrees with respect to the light source, the light detector measures substantially no direct light from the light source. The detected light is therefore substantially originating from the indicator micro-organisms or the light emitting substance they produce only, resulting in an improved measurement. It is noted that in any of the embodiments of the invention multiple light detectors and light sources can be provided.

Optionally, a light detector is provided at an angle of substantially 180 degrees with respect to the light source, i.e. a light detector is provided facing the light source. This enables a measurement of the light absorbed by the fluid sample which can be related to the total amount of indicator micro-organisms in the fluid sample.

Furthermore, an absorption measurement can serve as a calibration. The multiplication of indicator micro-organisms is influenced by various environmental conditions, such as temperature. The absorption measurement enables a measurement of the actual amount of indicator micro-organisms present in the fluid sample.

In a preferred embodiment according to the present invention the measurement means further comprise a light filter for filtering light of a specific wavelength and/or a mosaic light filter.

A mosaic light filter is an array of light filters of different color.

By providing a light filter in front of the light source, the fluid sample can be irradiated with light of a well-defined wavelength. This wavelength is determined by the filter. By providing a light filter in front of the light detector, the light detected comprises wavelengths in the wavelength region of interest only. This wavelength region is determined by the light filter.

An advantage of placing a light filter in front of the light source is that a band of wavelengths can be selected from the broad spectrum of the light source. For example, a red filter in front of a light source emitting white light will result in the irradiation of the fluid sample by red light.

An advantage of placing a light filter in front of the light detector is that those components of the light having wavelengths which are not in the wavelength region of interest can be filtered out. For example, in the case a red light emitting micro-organism or protein is used in combination with a green light emitting micro-organism or protein, the green light can be filtered out so that only the red light is detected.

The light filters are removable, such that for every specific application the appropriate filter can be selected. Preferably, changing light filters is handled automatically.

Instead of a single-colored light filter, a multi-colored light filter or a mosaic light filter can be used. This results in a multi-colored or full color image taken by the light detector. Subsequently, certain wavelengths from the detector image can be filtered out. Preferably, this is implemented as a software algorithm on a processing unit. For example, a full color image is obtained using the light detector, after which the blue and red components are selected using an algorithm implemented in software.

In a preferred embodiment according to the invention, the system comprises control means for controlling the light collection time of the light detector.

An advantage of controlling the light collection time of the light detector is that, in cases of low light intensities, the light collection time can be increased, such that eventually more light is collected and a better measurement results.

It is noted that the measures from the described embodiments can be combined in any combination. Furthermore, aspects of the analysis system can optionally be incorporated in the preparation module.

The invention further relates to a method for automatically preparing a fluid sample for toxicology analysis using an automatic toxicological fluid sample preparation module as described above.

The same effects and advantages apply in respect to such a method as those described in respect to the automatic toxicological fluid sample preparation module.

A preferred embodiment of the method according to the invention is defined in claim 9.

The preparation steps are selected on the basis of the specific application. The preparation steps can be programmed in an instruction set and for each specific application a different instruction set can be executed.

In a preferred embodiment according to the present invention, the method further comprises the step of flushing. Flushing improves the quality of the measurements.

Flushing comprises for example discharging a sample from the system to a waste container or cleaning the fluid chamber and/or the cuvette using a flushing fluid. This ensures that no residues are present during a measurement.

In a preferred embodiment according to the present invention the method further comprises:
- selecting two or more types of indicator micro-organisms which emit light, reflect light, absorb light and/or produce a light emitting substance, wherein the light is of the same or of different wavelength and its intensity is affected by one or more toxics and/or toxicological effects; and
- adding the two or more types of indicator micro-organisms to the fluid sample.

Adding two or more types of indicator micro-organisms enables the detection and quantification of more than one toxicological effect from a single fluid sample. For example, a membrane damaging effect and a DNA damaging effect can be detected.

In a preferred embodiment according to the present invention the method further comprises the steps of:
- locking the indicator micro-organisms in the log-phase of multiplication; and
- adding nutrition to the indicator micro-organisms before use.

Preferably, the indicator micro-organisms are locked in the log-phase of multiplication prior to being provided to the system. At room temperature, they can be kept in this state at least for months. When the micro-organisms are required for performing measurements, they are "unlocked" beforehand, for example by adding nutrition.

In a further preferred embodiment according to the present invention the method further comprises subjecting the indicator micro-organisms to light before use for stimulating multiplication of the micro-organisms.

By subjecting the indicator micro-organisms to light, they are stimulated to start multiplying. Preferably, green light is used with eukaryotic micro-organisms and red light is used with prokaryotic micro-organisms.

In a preferred embodiment according to the invention, the method further comprises performing measurements using a light detector and analyzing the measurements.

For example, this corresponds to a method for automatically preparing, measuring and analyzing a fluid sample using an automatic analysis system as described above.

In a preferred embodiment according to the present invention, the method further comprises the step of calibration.

Calibration ensures that the measurement and analysis are independent of internal and/or external influences. An example of an external influence is ambient temperature, which can influence the response of many detectors. Performing a calibration step prevents discrepancies between the actual situation and the results of the measurement and the analysis.

In a preferred embodiment according to the present invention, the method further comprises the steps of:
- adding fluorescent beads to the fluid sample before performing a calibration measurement;
- performing a calibration measurement by detecting the intensity of the light emitted by the fluorescent beads; and
- correcting measurements according to the relation between the light intensity detected in the calibration measurement and an expected light intensity.

The fluorescent beads emit light with a known intensity and with a known wavelength. By providing a filter in front of the detector, a measurement is performed in which only the wavelengths corresponding to the wavelength of the beads' fluorescent light are measured. The measured intensity is compared to the expected intensity of the light emitted by the fluorescent beads. This information is used for correction of measurements (subsequent, preceding or coincident with the calibration measurement).

In a preferred embodiment according to the present invention the method further comprises measuring a total amount of indicator micro-organisms.

The total amount of indicator micro-organisms can for example be measured by measuring the total absorption of the light with which the fluid sample is irradiated.

This enables, for example, to determine the ratio of healthy indicator micro-organisms and those who are affected by a toxic or toxicological effect. For example, indicator micro-organisms which emit bioluminescence light of a first wavelength are added to a fluid sample. Due to a toxicological effect, some of these indicator micro-organisms will emit less light. The amount of light with the first wavelength is measured in the measurement means. A second measurement is performed using a light source of second wavelength, being different than the first wavelength. The affected micro-organisms will still absorb the light, as will the healthy indicator micro-organisms. Therefore, the measurement means can quantify the amount of healthy organisms as a fraction of the total amount.

Further advantages, features and details of the invention are elucidated on the basis of preferred embodiments thereof, wherein reference is made to the accompanying drawings, in which:
fig. 1 shows a schematic representation of a first embodiment of an automatic toxicological analysis system according to the invention;
fig. 2 shows a 3D representation of the system of figure 1;
fig. 3 shows a different view of the automatic toxicological analysis system of fig. 2;
fig. 4 shows the upper part of the fluid sample preparation means of the system of figures 1-3, including a filter;
fig. 5 shows the measurement means according to the first embodiment;
fig. 6 shows a schematic representation of a second embodiment of an automatic toxicological analysis system according to the invention; and
fig. 7 shows a 3D representation of the embodiment of figure 6.

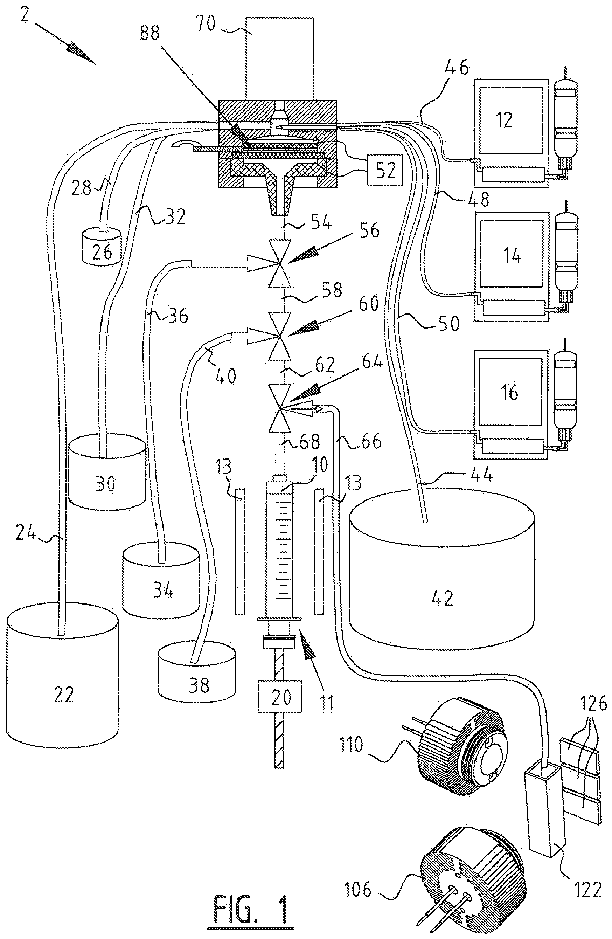

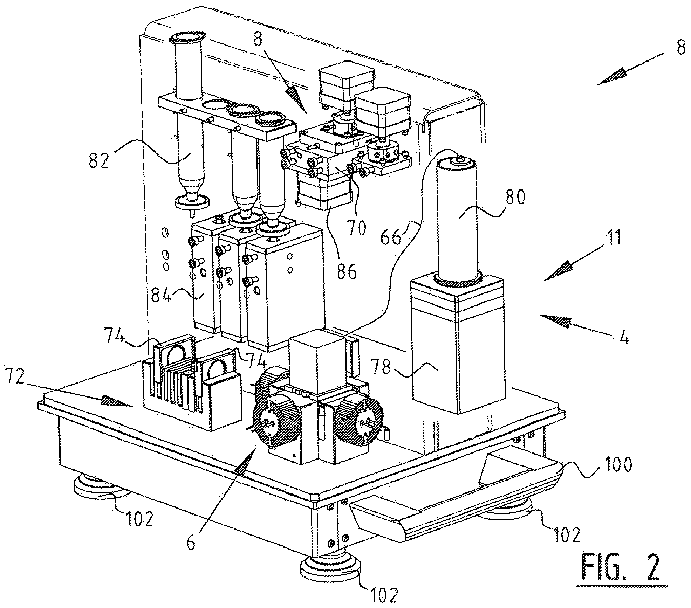

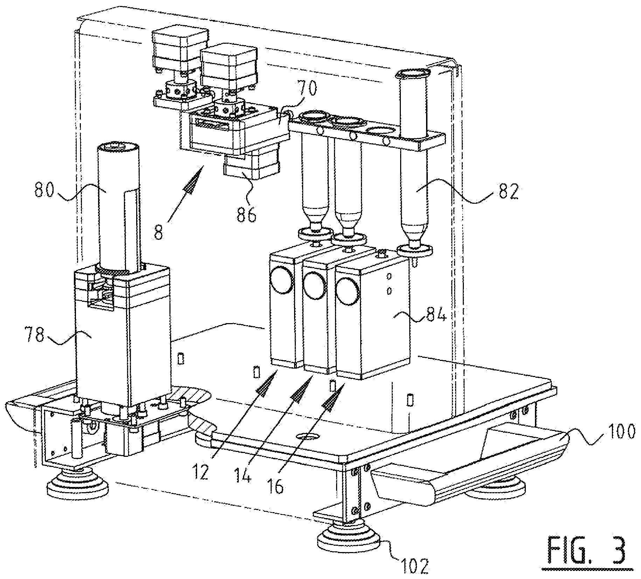

An automatic toxicological analysis system 2 comprises sample preparation means 4 and measurement means 6 (fig. 1, 2 and 3). In the illustrated embodiment, the fluid sample preparation means 4 and the measurement means 6 are provided as modular units.

The fluid sample preparation means comprise a filter with inlet and outlets 8, a fluid chamber 11, a piston 10, containers 12, 14, 16, 22, 26, 34 holding indicator micro-organisms, processing fluids and/or fluorescent beads, a waste container 42, connecting pipes or tubes 32, 40 for connecting the preparation means to the fluids or fluid streams 30, 38 to be analyzed. The filter part 8, comprising a filter 88, an inlet and an outlet, is connected to the fluid chamber 11 through a series of valves 56, 60, 64.

A sample from the fluids or fluid streams 30, 38 flows through a tube 32, 40, to the fluid preparation chamber 11. A solvent can be added from the solvent container 22 through the tube 24. Optionally, instead of a solvent container also a direct connector to a water tap can be provided. Furthermore, a displacement fluid can be obtained from a displacement fluid container 26 through tube 28.

A connection 32 to a first fluid or fluid stream 30 is provided. The filter part 8 is connected to a waste container 42 through connection 44. Containers 12, 14, 16 are connected to the filter part 8 through connections 46, 48, 50. Container 34 is connected to the filter part 8 and the fluid chamber 11 through connection 36 and valve 56. A second fluid or fluid stream 38 is connected to the filter part 8 and the fluid chamber 11 through connection 40 and valve 60. Valve 64 and connection 66 connect the sample preparation means to the measurement means 6.

The piston 10 is operated by a motor 20. By operating the piston 10, the valves 56, 60, 68 and pumps (not shown), the fluid streams inside the system can be controlled. The fluid sample preparation means further comprise heating and cooling elements 18 which in the illustrated embodiment comprise a Peltier element/ thermoelectric heat pump.

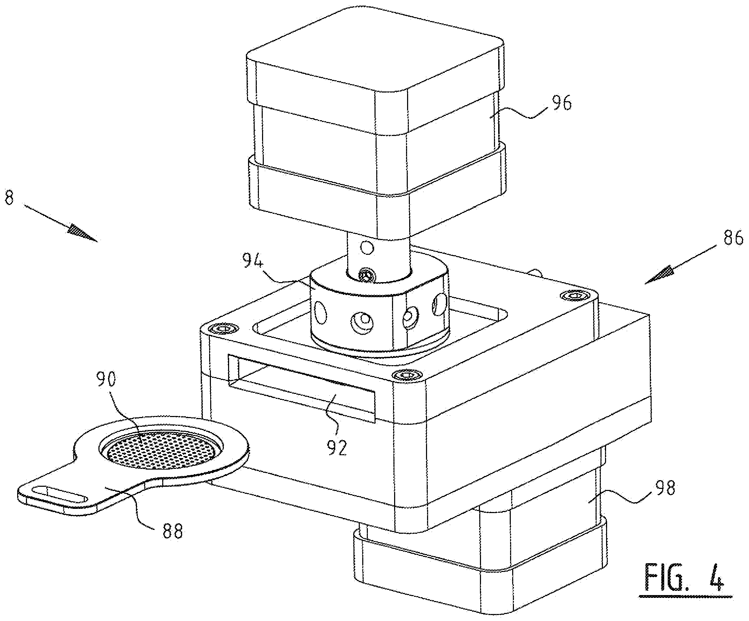

Fig. 4 shows a close up of the filter 88, which comprises holes 90. The filter can be removed or inserted into the filter part 8 through slot 92.

The measurement means 6 comprise a cuvette 122 comprising an inlet 128 for obtaining a fluid sample prepared by the fluid sample preparation means through connection 66. The cuvette 122 is situated in cavity 120.

The measurement means further comprise a light detector 126, which in this case is an array of CCD's. Further light detectors may be provided through holes 118, 119 or 121. The measurement means further comprise light source 104, 106, 110.

The measurement means further comprise light filters 74 which, when not in use, are placed in light filter container 72. If required, these filters can be placed in slots 200, 202 and/or 204. This can be performed manually or automatically.

Next, the system and the method according to the invention will be explained on the basis of the first embodiment according to the figures 1-5, in the context of an application in a water treatment facility.

The automatic analysis system 2 obtains a sample from fluids or fluid streams of the water treating process. In this case, a sample of treated water is obtained through connection 32 from fluid stream 30. The main goal in this example is to detect if any toxics are present in the fluids or fluid streams and, if this is the case, to give a quantification of their toxicological effect. By controlling the piston 10 with the motor 20 the sample is obtained from the source 30 through connections 32 to filter 88 and through connections 54, 58, 62 and 68 and valves 56, 60 and 68 into the fluid chamber 11.

Several preparation steps are performed. The steps of preparation are performed according to an instruction set which can be programmed in hardware or software (not shown). The one or more preparation steps are selected from the group comprising filtration, dilution, concentration, heating, cooling, mixing, pumping, adding a processing fluid, adding a micro-organism, adding nutrition and adding one or more fluorescent beads.

In the example of the water treatment facility, the first step comprises filtering out any micro-organisms which are present in the sample from the fluid or fluid stream 30, to allow a clean measurement. The sample flows from the source 30 through connection 32 and filter 88. Since the filter 88 comprises holes 90 of a diameter of 0.5 µm, which is the typical size of micro-organisms, it will filter out micro-organisms present in the sample of the fluid or fluid stream 30. The fluid sample flows subsequently to the fluid chamber 11. The micro-organisms retained by the filter are flushed in a later stage by pumping a displacement fluid from source 26 through connection 28 over the filter 88 to waste container 42 through connection 44.

In a preparation step indicator micro-organisms are added to the fluid sample from container 34, containing indicator micro-organisms in a glycerol environment, through connection 36 and valves 56, 60 and 64. In this example, the container 34 comprises a micropump for pumping a certain amount of indicator micro-organisms into the fluid chamber 11.

In this example, two types of indicator micro-organisms are added to the fluid sample by means of container 34 holding a first type and an additional container (not shown) holding a second type. The indicator micro-organisms of the first type obtain a red color in response to DNA damage and the indicator micro-organisms of the second type obtain a green color in response to a second toxicological effect.

The preparation means comprises mixing means for mixing the indicator micro-organisms with the fluid sample. They further comprise heating and cooling means 13, such as a Peltier element, for temperature treatment of the fluid sample, for example to stimulate growth of the indicator micro-organisms.

The fluid sample is now prepared and ready for the detection step. The piston 10 is activated with the motor 20 to push the fluid sample from the fluid chamber 11 through valve 68 and connection 66 to the measurement means 6.

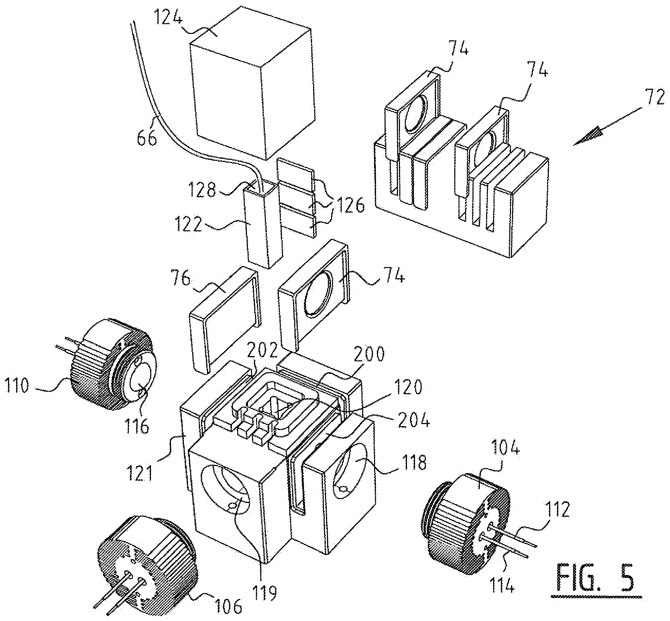

With reference to fig. 5, the fluid sample is transported to cuvette 122 through inlet 128. The measurement means comprise a light detector. In the embodiment of fig. 5 the light detector comprises an array of CCDs 126. Light emitting diodes 104 and 110 emit red and green light respectively.

Since the indicator micro-organisms of the first type obtain a red color when suffering from DNA damage, they can be detected using the red light. The green light is provided for the second type of indicator micro-organisms.

In the illustrated embodiments, the measurement of the red light and the green light are performed in separate steps. In a first measurement, a light filter 74 which blocks green light is placed in slot 200, so no green light will be detected by light detector 126. In a second measurement, a different light filter 74 which blocks red light is placed in slot 200. In the illustrated embodiment, the filters are interchanged automatically (not shown).

The intensity of the detected red and green light can be related to the quantity of indicator micro-organisms affected by the mutagen and second toxicological effect respectively. This requires a type of calibration, since the response of the measurement means may vary due to external and internal influences, such as ambient temperature. In this example, the calibration is performed using the fluorescent beads.

The fluorescent beads emit light of a known intensity and known wavelength, wherein the wavelength preferably differs from the wavelengths of the added indicator micro-organisms. It is possible that the beads require excitation by a light source provided in the measuring module 6 before they emit light. The fluorescent beads can comprise, for example, latex beads.

For example, the fluorescent beads can be obtained from containers 12, 14, 16, 26 and/or 34. The beads are added to the fluid sample after which a calibration step is performed. For example, they are added by the preparation means or manually. Furthermore adding means can be provided to add the beads to the cuvette of the measurement means.

The light emitted by the fluorescent beads is detected, wherein a light filter in front of the detector is used. The light filter blocks light of different wavelengths than the wavelength emitted by the beads. Therefore, only the light emitted by the beads is detected. The system is calibrated by relating the measured light intensity of the light emitted by the beads to the expected light intensity.

It is noted that the calibration measurement and the actual measurement can be performed at the same time if more than one detector is provided, wherein one detector detects the light emitted by the beads only. In this example, the calibration step and the actual measurement are performed subsequently.

In a third measurement using the same fluid sample, the total amount of indicator micro-organisms is measured using light source 106. The light source is provided facing detector 126.

In this measurement, the intensity of the detected light is related to the total light absorption by indicator micro-organisms of any type in the fluid sample.

It is noted that this measurement can be calibrated using a fluid sample which contains no indicator micro-organisms. This fluid sample can be prepared by obtaining a sample from source 30 through connection 32. This particular calibration can be performed before or after the measurements of the actual measurements.

The analysis step is performed automatically by a processing unit (not shown). In the example one of the analysis steps comprises calculation of the percentage of indicator micro-organisms affected by the mutagens and/or the second toxicological effect using the results from the third measurement in combination with the results of the first and/or second measurement.

A further analysis step comprises scaling the measurements to the results of the calibration measurements to obtain results which are independent of varying external and internal influences.

On the basis of the measurements it is for example possible to conclude that the amount of carcinogens in the water is unacceptably high. The system can send a control signal using the communication means, to interact with the process. In this case an alarm is raised and, if necessary, several valves can be closed so that the contaminated water does not leave the factory. The system further communicates the measurement and analysis results to an operator for informing the operator on the contamination and providing all the details.

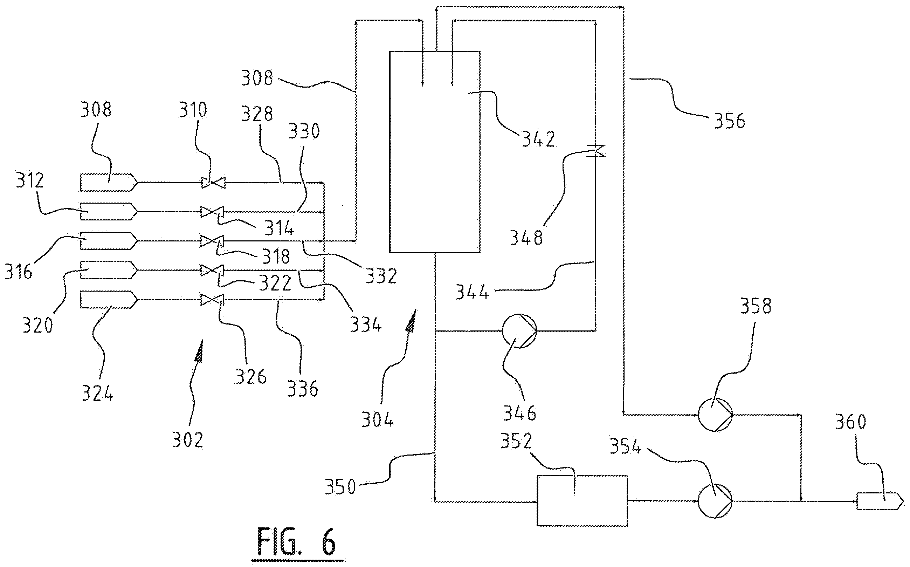

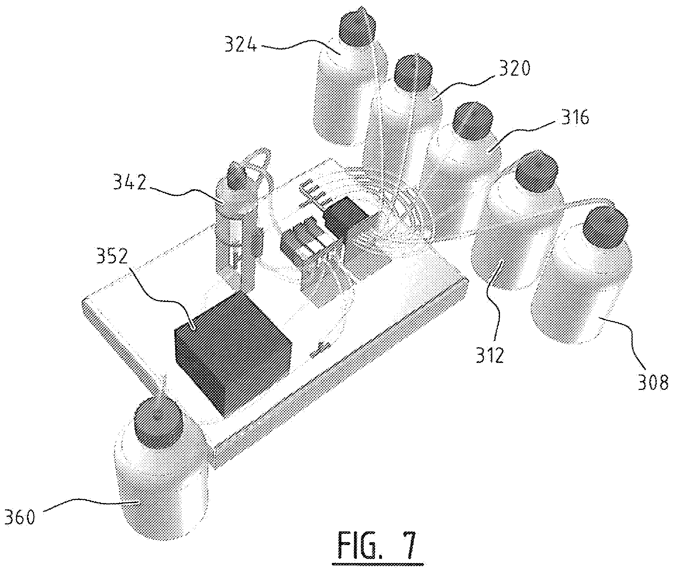

A second, currently preferred embodiment of the system according to the invention is schematically represented in figure 6 and shown in a 3D representation in figure 7.

The system comprises an input part 302, a reaction part 304 and a measurement part 306.

Input part 302 comprises a fluid stream 308 from which a sample to be analyzed can be obtained. The content of fluid stream 308 can be obtained by controlling valve 310. Alternatively, element 308 is a fluid container.

Input part 302 further comprises a container 312 containing a concentrated medium to stimulate growth of micro-organisms. Container 312 is connected to valve 314, which can be opened to obtain the medium.

Container 316 contains indicator micro-organisms, which can be fed to the system by opening valve 318.

Container 320 contains a buffer fluid, such as purified water, and is connected to valve 322.

Container 324 contains a disinfectant and is connected to valve 326.

Fluid stream 308 and containers 312, 316, 320, 324 are connected to reaction part 304 of the system via valves 310, 314, 318, 322, 326 and conduits 328, 330, 332, 334 and 336 respectively. The conduits connect to inlet 340 of reaction part 304.

Reaction part 304 comprises inlet 340 which is connected to reactor 342. The reactor 342 is connected to conduit loop 344, which further comprises pump 346 and heater 348 to circulate and heat the fluid from the reactor.

Reactor 342 is further connected to the measurement part 306 through conduit 350. Conduit 350 leads to detector 352 and pump 354.

Reactor 342 is furthermore connected to conduit 356 which comprises pump 358.

Both conduit 350 and conduit 356 are connected to waste container 360.

It is noted that no filter is required in this second embodiment.

A process for analyzing a fluid or fluid stream using the presently preferred embodiment as illustrated in figures 6-7 will be explained below in further detail.

In a first process step, the system is thoroughly cleaned. A disinfectant is obtained from container 324 by opening valve 326 and activating pump 346. Optionally, also a buffer fluid is obtained from container 320 by opening valve 322 to dilute the disinfecting fluid. Valves 322 and 326 are closed when a sufficient amount of disinfecting fluid is present in the system. Preferably, reactor 342 is completely filled with disinfecting fluid. The disinfectant is circulated in the loop comprising conduit 344 and reactor 342. Optionally, the disinfecting fluid is heated using heater 348.

After a certain period of time, pump 346 is switched off and pump 358 is switched on, to remove the disinfecting fluid from the system to waste container 360.

Next, the system is flushed with a buffer fluid from container 320, by controlling valve 322 and pump 346. Again, after a certain period of time, the fluid is discharged to waste container 360 by switching off pump 346 and switching on pump 358.

Optionally, the described steps can also be applied to disinfect and rinse measurement part 306. This can be achieved by controlling pump 354.

It is noted that it is possible to switch off all pumps in the system while the reactor 342 and/or conduit 344 and/or conduit 350 are completely filled with disinfecting fluid. In this state, the system is kept clean when it is not in use. Before starting a measurement, the system can discharge the disinfecting fluid and rinse the system using the buffer fluid as described above.

A measurement starts by obtaining indicator micro-organisms and growth medium from containers 316, 312 respectively, by controlling valves 318, 314 and pump 346. Optionally, also buffer fluid is added to reaction part 304 by controlling valve 322. The buffer fluid can dilute the growth medium.

The micro-organisms and the growth medium are mixed due to circulation via conduit 344. Preferably, the fluid is also heated using heater 348, to further stimulate multiplication of the indicator micro-organisms.

The mixture of micro-organisms, medium and possibly buffer fluid is circulated for a certain amount of time to enable multiplication of the micro-organisms. This period is typically about an hour, but could be less or more.

Subsequently, a sample of the fluid to be analyzed is obtained from fluid stream 308 and added to the fluid comprising indicator micro-organisms, by controlling valve 310 and pump 346. The fluid sample is circulated in the loop comprising conduit 344 and reactor 342 to enable interaction of the sample with the micro-organisms. Optionally, the heater 348 is activated to keep the fluid at a desired temperature.

Any toxics present in the sample fluid will interact with the micro-organisms during the so called "challenging phase". Typically, a period of half an hour is sufficient for a detectable effect of the sample fluid on the indicator micro-organisms. Shorter or longer periods can be used depending on the application.

After the "challenging phase", at least a part of the fluid is fed to detector 352 by controlling pump 354. After detection, the fluid is discharged to waste container 360.

An example of a micro-organism used for detecting a toxic substance is a micro-organism which comprises a lacZ gene, for instance an Escherichia coli bacteria. When the organism expresses the gene, a colourless substance is converted into a coloured substance. Therefore, if the micro-organism is alive, the coloured substance can be detected. However, when the expressing of the lacZ is affected by the interaction of a toxic and the micro-organism, less coloured substance will be produced. This is in indicator for DNA-damage.

It is noted that in the system and method according to the invention, also an alternative detector may be used instead of a detector using light, for instance a detector using the impedance of the fluid. Furthermore, the detector used in the first embodiment can be used in the second embodiment and vice versa.

## Claims

1. Automatic toxicological fluid sample preparation module (4) for automatically preparing a fluid sample for the detection and measurement of toxics and/or toxicological effects, the system comprising:
- an inlet (8) for automatically obtaining a sample directly from a fluid or fluid stream (30, 38, 308) to be analyzed;
- preparation means for preparing the fluid sample from the sample, comprising:
- first coupling means (32) for coupling with the inlet; and
- a first inlet (340) for obtaining a sample from a fluid or fluid stream to be analyzed;
- a fluid chamber (11, 342) connected to the first inlet;
- fluid sample preparation means comprising one or more of the following components: a filter (88), a heater (348), a cooler, a pump (346), a piston and mixing means; and
- a first outlet for discharging a prepared fluid sample, the first outlet being connectable to the measurement means; and
- an outlet (8) for discharge of prepared fluid to measurement means,
wherein the preparation means comprise indicator micro-organisms, **characterized in that** the module comprises a conduit loop (344) for circulating the fluid sample in the loop to enable interaction of the sample with the indicator micro-organisms, and further **characterized in that** the indicator micro-organisms emit light and/or produce a light emitting substance, intrinsically and/or in response to a toxic and/or toxicological effect and wherein the indicator micro-organisms are locked in the log-phase of multiplication before use
wherein the preparation means further comprise nutrition for micro-organisms for adding the nutrition to the indicator micro-organisms, and
wherein the preparation means further comprise a light source (104, 106, 110) for irradiation of the indicator micro-organisms, wherein the light source emits light of a wavelength which stimulates the multiplication of micro-organisms, and wherein the automatic toxicological fluid sample preparation module further comprises killing means for killing the indicator micro-organisms.

2. Automatic toxicological fluid sample preparation module according to claim 1, comprising communication means.

3. Automatic toxicological fluid sample preparation module according to one or more of the claims 1-2, wherein the fluid sample preparation means comprise a modular container system, comprising one or more containers (12, 14, 16, 34, 312, 316, 320, 324) for holding processing fluids, indicator micro-organisms, nutrition, and/or fluorescent beads.

4. Automatic toxicological analysis system comprising the fluid sample preparation module according to one or more of the claims 1-3 and a second module comprising measurement means, preferably comprising a light detector.

5. Automatic toxicological analysis system according to claim 4, the measurement means comprising:
- a light detector (126); and
- a cuvette (122) with a length,
wherein the cuvette comprises:
- an inlet (128) for obtaining the fluid sample, and
- an outlet for discharging the fluid sample,
preferably wherein one or more of the light detectors comprises a Charge Coupled Device provided in an array extending substantially over the length of the cuvette.

6. Automatic toxicological analysis system according to claim 4 or 5, wherein the measurement means further comprise a light source, wherein the light detector preferably is placed at an angle of substantially 90 degrees with respect to the light source.

7. Automatic toxicological analysis system according to claim 5, 6 or 7, further comprising control means for controlling the light collection time of the light detector.

8. Method for automatically preparing a fluid sample for toxicology analysis using an automatic toxicological fluid sample preparation module according to one or more of the claims 1-3.

9. Method according to claim 8, comprising the steps of:
- obtaining a sample directly and automatically from a fluid or fluid stream (30, 38, 308) to be analyzed;
preparing the fluid sample from the sample by selecting one or more preparation steps from the group comprising filtration, dilution, concentration, heating, cooling, mixing, pumping, adding a processing fluid and adding indicator micro-organisms; and further comprising the steps of:
- selecting two or more types of indicator micro-organisms which emit light, reflect light, absorb light and/or produce a light emitting substance, wherein the light is of the same or of different wavelength and its intensity is affected by one or more toxics and toxicological effects; and
- adding the two or more types of indicator micro-organisms to the fluid sample; and comprising the steps of:
- locking the indicator micro-organisms in the log-phase of multiplication; and
- adding nutrition to the indicator micro-organisms.

10. Method according to one or more of the claims 8-9, further comprising the step (126) of performing measurements using a light detector and analyzing the measurements, preferably further comprising the steps of:
- adding fluorescent beads to the fluid sample before performing a calibration measurement;
- performing the calibration measurement by measuring the intensity of the light emitted by the fluorescent beads; and
- correcting measurements according to the relation between the light intensity measured in the calibration measurement and an expected light intensity; and
- measuring a total amount of indicator micro-organisms in the fluid sample; and
- adapting micro-organisms to obtain indicator micro-organisms.

## Patentansprüche

1. Modul zur automatischen toxikologischen Flüssigkeitsprobennahme (4) zum automatischen Erstellen einer Flüssigkeitsprobe zum Erfassen und Messen von Schadstoffen und/oder toxikologischen Effekten, wobei das System aufweist:
- einen Einlass (8), um eine Probe direkt von einer zur analysierenden Flüssigkeit oder einem Flüssigkeitsfluss (30, 38, 308) zu erhalten;
- Erstellungsmittel zum Erstellen der Flüssigkeitsprobe aus der Probe, die aufweisen:
- erste Kopplungsmittel (32) zum Koppeln mit dem Einlass; und
- einen ersten Einlass (340), um eine Probe von einer zur analysierenden Flüssigkeit oder einem Flüssigkeitsfluss zu erhalten;
- eine Flüssigkeitskammer (11, 342), die mit dem ersten Einlass verbunden ist;
- Herstellungsmittel einer Flüssigkeitsprobe, die eine oder mehrere der folgenden Komponenten aufweisen: einen Filter (88), eine Heizvorrichtung (348), eine Abkühlungsvorrichtung, eine Pumpe (3 46), einen Kolben und Mischmittel; und
- einen ersten Auslass, um eine erstellte Flüssigkeitsprobe abzulassen, wobei der erste Auslass mit Messmitteln verbindbar ist; und
- einen Auslass (8), um die hergestellte Flüssigkeit in Messmittel abzulassen, wobei die Erstellungsmittel Indikator-Mikroorganismen aufweisen, **dadurch gekennzeichnet, dass**
das Modul einen Leitungskreis (344) aufweist, um die Flüssigkeitsprobe in dem Kreislauf zu zirkulieren, um eine Interaktion der Probe mit den Indikator-Mikroorganismen zu ermöglichen, und
weiterhin **dadurch gekennzeichnet, dass**
die Indikator-Mikroorganismen Licht ausstrahlen und/oder eine lichtausstrahlende Substanz intrinsisch und/oder in Reaktion auf einen Schadstoff und/oder toxikologischen Effekt erzeugen, und wobei die Indikator-Mikroorganismen in der logarithmischen Phase der Multiplikation vor der Verwendung gesperrt sind,
wobei die Herstellungsmittel weiterhin Nährstoffe für die Mikroorganismen aufweisen, um die Nährstoffe zu den Indikator-Mikroorganismen hinzuzufügen, und
wobei die Erstellungsmittel weiterhin eine Lichtquelle (104, 106, 110) zum Bestrahlten der Indikator-Mikroorganismen aufweisen, wobei die Lichtquelle Licht einer Wellenlänge ausstrahlt, die die Multiplikation der Mikroorganismen anregt, und wobei das Modul zur automatischen toxikologischen Flüssigkeitsprobennahme weiterhin Abtötungsmittel zum Abtöten der Indikator-Mikroorganismen aufweist.

2. Modul zur automatischen toxikologischen Flüssigkeitsprobennahme nach Anspruch 1, das weiterhin Kommunikationsmittel aufweist.

3. Modul zur automatischen toxikologischen Flüssigkeitsprobennahme nach einem oder mehreren der Ansprüche 1 bis 2, wobei die Mittel zur Flüssigkeitsprobennahme ein modulares Behältersystem aufweisen, das einen oder mehrere Behälter (12, 14, 16, 34, 312, 316, 320, 324) aufweist, um die Verarbeitungsflüssigkeiten, Indikator-Mikroorganismen, die Nährstoffe und/oder die fluoreszierenden Kügelchen zu enthalten.

4. System zur automatischen toxikologischen Analyse, das das Modul zur Flüssigkeitsprobennahme nach einem oder mehreren der Ansprüche 1 bis 3 und ein zweites Modul aufweist, das Messmittel, vorzugsweise einen Lichtdetektor aufweist.

5. System zur automatischen toxikologischen Analyse nach Anspruch 4, wobei die Messmittel aufweisen:
- einen Lichtdetektor (126); und
- eine Küvette (122) mit einer Länge,
wobei die Küvette aufweist:
- einen Einlass (128), um die Flüssigkeitsprobe zu erhalten, und
- einen Auslass, um die Flüssigkeitsprobe abzulassen,
wobei vorzugsweise einer oder mehrere der Lichtdetektoren eine ladungsgekoppelte Vorrichtung aufweisen, die in einem Array vorgesehen ist, der sich im Wesentlichen über die Länge der Küvette erstreckt.

6. System zur automatischen toxikologischen Analyse nach Anspruch 4 oder 5, wobei die Messmittel weiterhin eine Lichtquelle aufweisen, wobei der Lichtdetektor vorzugsweise in einem Winkel von im Wesentlichen 90° bezüglich der Lichtquelle angeordnet ist.

7. System zur automatischen toxikologischen Analyse nach Anspruch 5, 6 oder 7, das weiterhin Steuermittel aufweist, um die die Lichterhebungszeit des Lichtdetektors zu steuern.

8. Verfahren zum automatischen Erstellen einer Flüssigkeitsprobe für eine toxikologische Analyse mittels eines Moduls zur automatischen toxikologischen Flüssigkeitsprobennahme nach einem der Ansprüche 1 bis 3.

9. Verfahren nach Anspruch 8, das die Schritte aufweist:
- Erhalten einer Probe direkt und automatisch von einer zur analysierenden Flüssigkeit oder eines Flüssigkeitsflusses (30, 38, 308);
Erstellen der Flüssigkeitsprobe aus der Probe, indem ein oder mehrere Herstellungsschritte aus der Gruppe ausgewählt werden, die die aufweist: Filterung, Verdünnung, Konzentration, Erwärmen, Abkühlen, Mischen, Pumpen, Hinzufügen einer Verarbeitungsflüssigkeit und Hinzufügen von Indikator-Mikroorganismen; und
weiterhin die Schritte aufweist:
- Auswählen von zwei oder mehreren Arten von Indikator-Mikroorganismen, die Licht ausstrahlen, Licht reflektieren, Licht absorbieren und/oder eine lichtausstrahlende Substanz erzeugen, wobei das Licht von der gleichen oder einer anderen Wellenlänge ist und die Intensität von einem oder mehreren Schadstoffen und toxikologischen Effekten beeinflusst wird; und
- Hinzufügen der zwei oder mehreren Arten von Indikator-Mikroorganismen zur Flüssigkeitsprobe; und
die Schritte aufweist:
- Sperren der Indikator-Mikroorganismen in der logarithmischen Phase der Multiplikation; und
- Hinzufügen von Nährstoffen zu den Indikator-Mikroorganismen.

10. Verfahren nach einem oder mehreren der Ansprüche 8 bis 9, die weiterhin den Schritt (126) des Durchführens von Messungen mittels eines Lichtdetektors und der Analyse der Messungen aufweist, das vorzugsweise weiterhin die Schritte aufweist:
- Hinzufügen von fluoreszierenden Kügelchen zu der Flüssigkeitsprobe vor Durchführung einer Kalibrierungsmessung;
- Durchführen der Kalibrierungsmessung durch Messen der Intensität des von den fluoreszierenden Kügelchen ausgestrahlten Lichts; und
- Korrigieren von Messungen bezüglich der Beziehung zwischen der in der Kalibrierungsmessung gemessenen Lichtintensität und einer erwarteten Lichtintensität; und
- Messen einer Gesamtmenge von Indikator-Mikroorganismen in der Flüssigkeitsprobe; und
- Anpassen der Mikroorganismen, um Indikator-Mikroorganismen zu erhalten.

## Revendications

1. Module de préparation automatique d'échantillon de fluide toxicologique (4) pour préparer automatiquement un échantillon de fluide pour la détection et la mesure de produits toxiques et/ou d'effets toxicologiques, le système comprenant :
- un orifice d'entrée (8) pour obtenir automatiquement un échantillon directement en provenance d'un fluide ou flux de fluide (30, 38, 308) à analyser ;
- des moyens de préparation pour préparer l'échantillon de fluide à partir de l'échantillon, comprenant :
- des premiers moyens d'accouplement (32) pour un accouplement à l'orifice d'entrée ; et
- un premier orifice d'entrée (340) pour obtenir un échantillon en provenance d'un fluide ou flux de fluide à analyser ;
- une chambre de fluide (11, 342) raccordée au premier orifice d'entrée ;
- des moyens de préparation d'échantillon de fluide comprenant un ou plusieurs des composants suivants : un filtre (88), un élément chauffant (348), un refroidisseur, une pompe (346), un piston et des moyens de mélange ; et
- un premier orifice de sortie pour évacuer un échantillon de fluide préparé, le premier orifice de sortie pouvant être raccordé aux moyens de mesure ; et
- un orifice de sortie (8) pour l'évacuation de fluide préparé vers des moyens de mesure,
dans lequel les moyens de préparation comprennent des microorganismes indicateurs, **caractérisé en ce que** le module comprend une boucle de conduit (344) pour faire circuler l'échantillon de fluide dans la boucle pour permettre une interaction de l'échantillon avec les microorganismes indicateurs,
et **caractérisé en outre en ce que**
les microorganismes indicateurs émettent une lumière et/ou produisent une substance électroluminescente, intrinsèquement et/ou en réponse à un produit toxique et/ou un effet toxicologique et dans lequel les microorganismes indicateurs sont bloqués dans la phase logarithmique de multiplication avant leur utilisation
dans lequel les moyens de préparation comprennent en outre une nutrition pour des microorganismes pour ajouter la nutrition aux microorganismes indicateurs, et
dans lequel les moyens de préparation comprennent en outre une source de lumière (104, 106, 110) pour une irradiation des microorganismes indicateurs, dans lequel la source de lumière émet une lumière d'une longueur d'onde qui stimule la multiplication de microorganismes, et dans lequel le module de préparation automatique d'échantillon de fluide toxicologique comprend en outre des moyens de destruction pour détruire les microorganismes indicateurs.

2. Module de préparation automatique d'échantillon de fluide toxicologique selon la revendication 1, comprenant des moyens de communication.

3. Module de préparation automatique d'échantillon de fluide toxicologique selon une ou plusieurs des revendications 1 et 2, dans lequel les moyens de préparation d'échantillon de fluide comprennent un système de contenants modulaires, comprenant un ou plusieurs contenants (12, 14, 16, 34, 312, 316, 320, 324) pour contenir des fluides de traitement, des microorganismes indicateurs, une nutrition, et/ou des billes fluorescentes.

4. Système d'analyse de toxicologie automatique comprenant le module de préparation d'échantillon de fluide selon une ou plusieurs des revendications 1 à 3 et un second module comprenant des moyens de mesure, comprenant de préférence un détecteur de lumière.

5. Système d'analyse de toxicologie automatique selon la revendication 4, les moyens de mesure comprenant :
- un détecteur de lumière (126) ; et
- une cuvette (122) avec une longueur,
dans lequel la cuvette comprend :
- un orifice d'entrée (128) pour obtenir l'échantillon de fluide, et
- un orifice de sortie pour évacuer l'échantillon de fluide,
dans lequel de préférence un ou plusieurs des détecteurs de lumière comprennent un dispositif à couplage de charge prévu dans une barrette s'étendant sensiblement sur la longueur de la cuvette.

6. Système d'analyse de toxicologie automatique selon la revendication 4 ou 5, dans lequel les moyens de mesure comprennent en outre une source de lumière, dans lequel le détecteur de lumière est de préférence placé à un angle de sensiblement 90 degrés par rapport à la source de lumière.

7. Système d'analyse de toxicologie automatique selon la revendication 5, 6 ou 7, comprenant en outre des moyens de régulation pour réguler le temps de captage de lumière du détecteur de lumière.

8. Procédé de préparation automatique d'un échantillon de fluide pour une analyse de toxicologie à l'aide d'un module de préparation automatique d'échantillon de fluide toxicologique selon une ou plusieurs des revendications 1 à 3.

9. Procédé selon la revendication 8, comprenant les étapes de :
- obtention d'un échantillon directement et automatiquement depuis un fluide ou flux de fluide (30, 38, 308) à analyser ;
préparation de l'échantillon de fluide à partir de l'échantillon en sélectionnant une ou plusieurs étapes de préparation dans le groupe comprenant la filtration, la dilution, la concentration, le chauffage, le refroidissement, le mélange, le pompage, l'ajout d'un fluide de traitement et l'ajout de microorganismes indicateurs ; et comprenant en outre les étapes de :
- sélection de deux types ou plus de microorganismes indicateurs qui émettent de la lumière, réfléchissent la lumière, absorbent la lumière et/ou produisent une substance électroluminescente, dans lequel la lumière a la même longueur d'onde ou une longueur d'onde différente et son intensité est affectée par un ou plusieurs produits toxiques et effets toxicologiques ; et
- ajout des deux types ou plus de microorganismes indicateurs à l'échantillon de fluide ; et comprenant les étapes de :
- blocage des microorganismes indicateurs dans la phase logarithmique de multiplication ; et
- ajout de nutrition aux microorganismes indicateurs.

10. Procédé selon une ou plusieurs des revendications 8 et 9, comprenant en outre l'étape (126) de réalisation de mesures à l'aide d'un détecteur de lumière et d'analyse des mesures, comprenant en outre de préférence les étapes de :
- ajout de billes fluorescentes à l'échantillon de fluide avant de réaliser une mesure d'étalonnage ;
- réalisation de la mesure d'étalonnage en mesurant l'intensité de la lumière émise par les billes fluorescentes ; et
- correction de mesures selon la relation entre l'intensité de lumière mesurée lors de la mesure d'étalonnage et une intensité de lumière attendue ; et
- mesure d'une quantité totale de microorganismes indicateurs dans l'échantillon de fluide ; et
- adaptation de microorganismes pour obtenir des microorganismes indicateurs.
